# EUROPEAN PATENT APPLICATION

(11) **EP 4 194 851 A1**
(43) Date of publication of application: **14.06.2023**
(21) Application number: 21212700.5
(22) Date of filing: 07.12.2021
(51) Int. Cl.: G01N 33/00

(54) **ELECTRONIC ENVIRONMENT MONITORING SYSTEM AND MONITORING METHOD THEREOF FOR SUPPLYING THE COLLECTED DATA**

(71) Applicant: Weargraph S.r.l., 20159 Milano (MI) (IT)
(72) Inventor: POZZONI, Alfio, 20159 Milano (MI) (IT)
(74) Representative: Burchielli, Riccardo

(57) **Abstract**

Described is an electronic system for monitoring the environment and, in particular, for monitoring substances present in the air or water, comprising a smartphone (10) in use by a user (1) and a device (20) wearable by the user (1), which is equipped with an electronic circuit (30) with relative electronic board (31) and CPU (32), a wireless communication chip (33), environmental sensors (21, 22, 23), a series of management chips of the environmental sensors (34, 35, 36), a mass memory (37) and a rechargeable battery (40). The environmental sensors (21, 22, 23) are configured to collect (50) a series of environmental data, which are acquired (51), via wireless technology, by a software application or APP (11) present in the smartphone (10) in use by the user (1), which is configured to geolocate the acquired data (51).

## Description

This invention relates to an electronic system for the mobile detection of substances present in the air or in water and comprises two electronic devices (a smartphone and a wearable electronic device) to interact, through a dedicated software application (APP) operating on the smartphone, with a series of databases inserted in a shared and immutable data structure (in particular, a platform with blockchain technology).

The wearable electronic device can be fixed to a user's jacket, shirt and/or trousers using a Velcro^{®} strip, a button or a clip and has a series of dedicated sensors that allow detection, for example in the case of detection of substances present in the air, of the atmospheric characteristics of the location where the user is present.

The above-mentioned wearable electronic device operates, according to the invention, with the smartphone also in asynchronous mode, thanks to its own local memory which is able to overcome any possible and temporary absence of the connection to the smartphone and comprises an electronic circuit with relative electronic card and CPU, a communication and wireless transmission chip, a series of chips for management of various environmental sensors, a mass memory and a rechargeable battery.

The same wearable electronic device, again in the case of detection of substances present in the air, has at least three environmental sensors for detecting the ambient temperature, the degree of humidity and the possible presence of physical, chemical and biological agents in the environment (in particular those relating to air pollution, such as PM10/PM12).

The data collected by the environmental sensors are acquired via wireless technology by the software application (APP) installed in the user's smartphone, which geo-locates them.

The software application (APP) allows the user to view the collected data and send them to a third-party organization, which has one or more platforms with shared and immutable data structures, which uses/does not use blockchain technology, in order to guarantee the security of both the environmental data acquired and the data of the user who sent them (the data are stored in special databases).

In particular, with regard to the security of the data of the system's user and in compliance with current privacy regulations and the GDPR, these personal data will be managed by the third-party organization uncoupled between the user's personal information, which are confidential and are stored in a database without external access, and the environmental data acquired by it, which are stored in other databases and therefore managed anonymously by the platform and by other users.

The organization that owns the platform then sells, after definition with the user who acquires and supplies the environmental data of a specific contract, solely the geo-located environmental data to other third parties (for example, entities and/or organizations and/or public or private companies), which use the above-mentioned environmental data for their specific activities.

The third parties pay the environmental data acquired to the organization, which converts the collection into "utility tokens", which are associated in a secure and certain manner to the user of the electronic system for the mobile detection of substances present in the air or in water that made them available.

The user, again through the software application (APP) and once appropriately recognized by the platform based on the relative credentials, can consult the data to which the relative credentials provide access, including the quantity of "utility tokens" assigned; the "utility tokens" can be requested and subsequently used for purchases on various channels that accept this type of "utility token".

The same user or other users who have the same electronic system for the mobile detection of substances present in the air or in water, according to the invention, can request from the platform of the third-party organization, again through the software application (APP), to have the environmental data of the place in which they are located or where they intend to move to and will be able to view or receive service communications from the platform, including alerts, for services belonging to the organization that owns the platform or its partners, always providing that the user has made an explicit request or has accepted the proposals sent.

The number of people traveling for education, work, sport, tourism, etc. has increased in our society.

During these movements, the individuals, if equipped with this electronic system for the mobile detection of substances present in the air or in water, according to the invention, can become "mobile observers" to acquire important data relating to the quality of the environment and the possible presence of pollutants, increasing the amount of data relating to the environment, which are now collected only by a few fixed control units, usually located in urban centres and almost non-existent in the suburbs and/or in open areas, which are zones often frequented by people and/or sportspersons and/or tourists.

The aim of the invention is therefore to overcome the above-mentioned technical drawbacks and, in particular, to provide a new system and method for collecting environmental data on the ground surface (e.g. people walking at low level or in mountains), underground (e.g. cavers), surface water (e.g. swimmers, sailors, surfers), deep sea (e.g. divers) and in the air (e.g. hang gliders, parachutists), which are able to expand the quantity of environmental data at the basis of technical-scientific studies which are indispensable for deciding public order actions, such as, for example, the blocking of traffic in a specific geographical area or urban centre.

It is therefore possible to create a network of "mobile observatories", which, equipped with the wearable electronic device with the dedicated sensors according to the invention, can detect many environmental data sets, for example in the case of detection of substances present in the air (temperature, humidity, pollution rate and/or physical, chemical and biological agents), of several areas and/or locations, in order to allow specialized companies to process them to suggest actions of physical protection for the community.

Another aim of the invention is to broaden the sense of necessary environmental protection, which is one of the most demanding challenges for the present and future human generations, with direct and easily implemented actions thanks to the digital development already available. A further aim of the invention is to have the environmental data for your location at your disposal, allowing you to know if that location is "healthy" for carrying out sports activities in safety and/or suitable or not for less strong individuals (children and the elderly).

Further characteristics and advantages of the invention will become more evident from the following description of a preferred but non-limiting embodiment of a method of detecting substances present in the air, with reference to the following drawings:

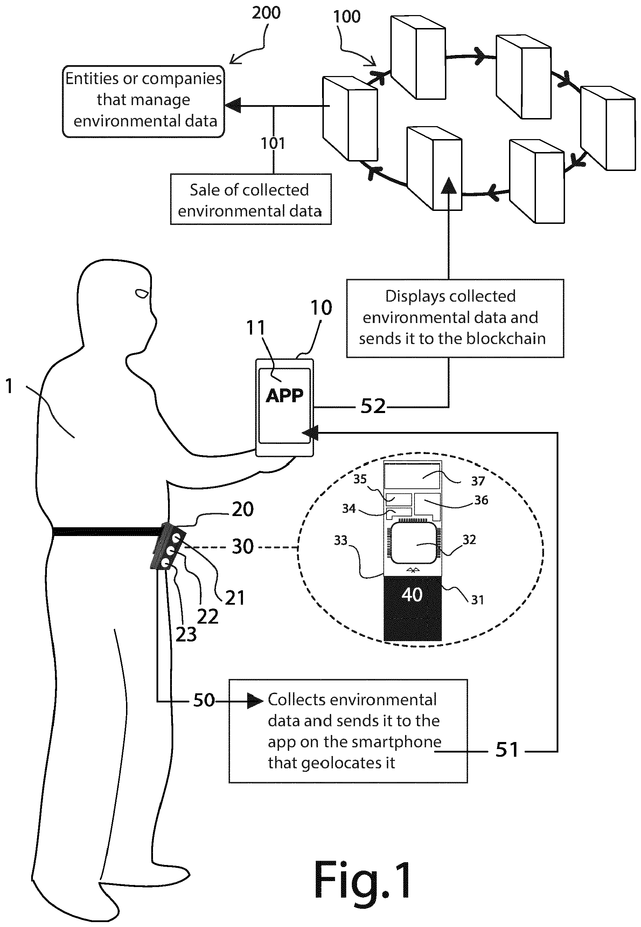

- Figure 1 shows the view of a smartphone user, with the software application (APP) for the management of the electronic system for the mobile detection of the substances present, in this case in the air, which communicates with the specific wearable electronic device for the collection of atmospheric data (temperature, humidity, pollution rate and/or physical, chemical and biological agents) and the subsequent activities of sending the data collected securely to a platform of a third-party organization with blockchain technology, which sells them to third parties who use the above-mentioned environmental data for their specific activities, according to the invention;

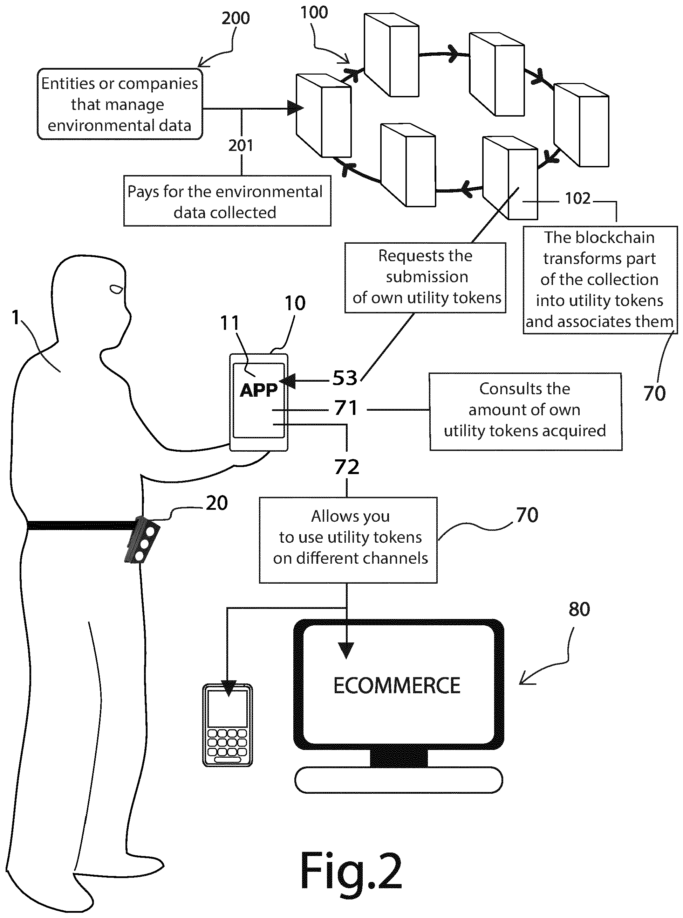

- Figure 2 shows the view of a smartphone user, with the software application (APP) for the management of the electronic system for the mobile detection of the substances present, in this case in the air, which communicates with the platform with blockchain technology, which converts the collection of the data received by the user and sold to third parties into "utility tokens" and makes them available to the user for subsequent purchases through various channels that accept them, according to the invention:

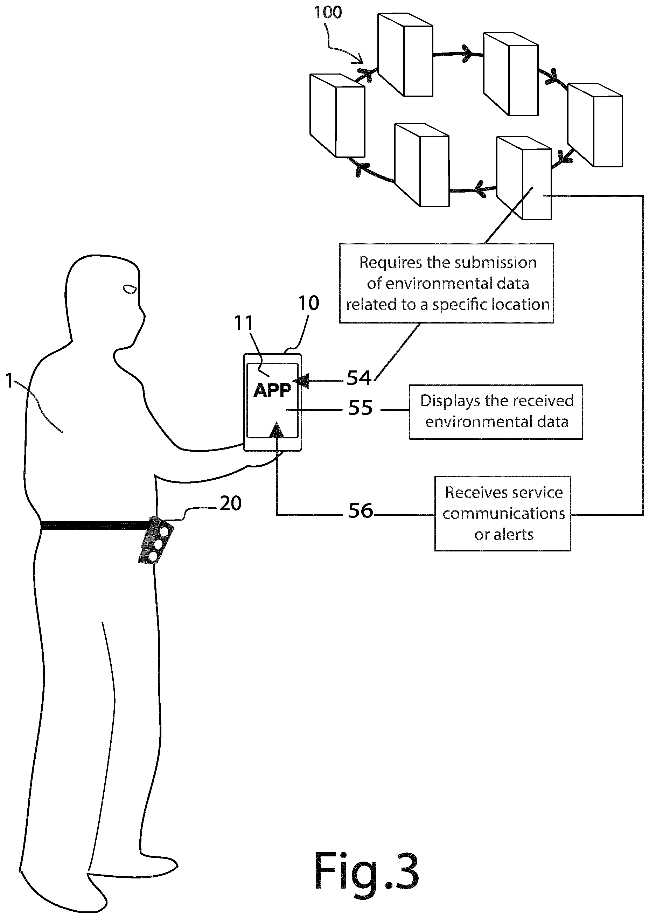

- Figure 3 shows the view of a smartphone user, with the software application (APP) for the management of the electronic system for the mobile detection of the substances present, in this case in the air, which communicates with the platform with blockchain technology to request and receive the environmental data of the relative location and/or other service communications, in order to know the air quality of the location, according to the invention.

With reference to the above-mentioned drawings, the electronic system according to the invention for the mobile detection of substances present in the air or in water, for which, specifically, the case of detection of substances present in the air is illustrated, comprises a device (20) wearable by a user (1), equipped with an electronic circuit (30) with relative electronic card (31) and CPU (32), a Bluetooth or other type of wireless transmission communication chip (33), a series of management chips of various environmental sensors (34, 35, 36), a mass memory (37) and a rechargeable battery (40).

The same wearable electronic device (20) has at least three environmental sensors for detecting the ambient temperature (21), the degree of humidity (22) and the possible presence of physical, chemical and biological agents in the air (23) and, in particular, those relating to air pollution.

The data collected (50) by the environmental sensors are acquired (51), via wireless technology, by a software application or APP (11) present for the purpose in the smartphone (10) used by the user (1) which geo-locates them.

The software application or APP (11) allows the user (1) to view the collected data (51) and to send them (52) to a platform (100) of a third party organization, which stores them on various databases inserted in a relative shared and immutable data structure, which uses blockchain technology, in order to guarantee the security of both the environmental data acquired and the data of the user (1) who sent them.

In particular, the personal data of the user (1) are managed securely in a database without external access by other third parties, while the environmental data acquired by them are stored in other databases and therefore managed anonymously by the platform (100) and other third party companies.

The organization that owns the platform (100) then sells (101) solely the geo-located environmental data to third parties (200), such as, for example, entities and/or organizations and/or public or private companies, which use the above-mentioned geo-located environmental data for their specific activities.

The third parties (200) pay (201) the environmental data acquired from the organization, which converts the data obtained (102) into "utility tokens" (70), which are associated in a secure and certain manner to the user (1) who is the supplier of the environmental data.

The user (1), duly recognized by the platform (100) through the software application (APP) (11) present on the relative smartphone (10) and relative access credentials, can consult and view (71) the quantity of associated "utility token" (70), request them (53) and then use them (72) for purchases on various channels, such as for example shops and/or e-commerce sites (80) that accept this type of "utility token" (70).

The same user (1) or other users who have the electronic system for the mobile detection of the substances present in the air or in water, according to the invention, can also request (54) from the platform (100), again through the software application (11) and relative access credentials to obtain the environmental data of a specific location or area and will be able to view (55) or receive (56) from the platform (100) service communications, including alerts, for relative services of the organization that owns the platform or its partners, provided that the user has made an explicit request or has accepted the proposals sent.

The invention described can be modified and adapted in several ways without thereby departing from the scope of the inventive concept of the accompanying claims.

Moreover, all the details can be replaced by other technically-equivalent elements.

Lastly, the components used, providing they are compatible with the specific use, as well as the dimensions, may vary according to requirements and the state of the art.

Where the features and the techniques mentioned in the following claims are followed by reference signs, the reference signs have been used only with the aim of increasing the intelligibility of the claims themselves and, consequently, the reference signs do not constitute in any way a limitation to the interpretation of each element identified, purely by way of example, by the reference signs.

## Claims

1. Electronic system for monitoring the environment and, in particular, for monitoring substances contained in air or water, **characterized in that** said system comprises a smartphone (10) of a user (1) and a wearable device (20) worn by said user (1), said wearable device (20) being equipped with an electronic circuit (30) with an electronic card (31), a CPU (32), a wireless communication chip (33), environmental sensors (21, 22, 23), a series of management chips for controlling said environmental sensors (34, 35, 36), a mass memory (37) and a rechargeable battery (40), said environmental sensors (21, 22, 23) being configured for collecting (50) a series of environment data, which are acquired (51), via wireless technology, from a software APP (11) running on said smartphone (10) of said user (1), said software APP (11) also being configured to geo-locate said environment acquired data (51).

2. Electronic system as claimed in claim 1, **characterized in that** said environmental sensors (21, 22, 23) are configured to detect terrestrial and/or underground and/or altitude environmental atmospheric data, such as the ambient temperature (21), the humidity (22) and the presence of physical, chemical and biological substances in the air (23), or environmental water data, such as the surface temperature (21), the salinity degree (22), the presence of physical, chemical and biological substances in the water (23).

3. Electronic system as claimed in at least one of the preceding claims, **characterized in that** said software APP (11) is configured to display said environment acquired data (51) on said smartphone (10) of said user (1) and so as to send said environment acquired data (52) to a digital platform (100) belonging to third-party companies, which stores said data on a plurality of databases.

4. Electronic system as claimed in claim 3, **characterized in that** said databases are inserted in a data structure using a block-chain technology, in order to guarantee the security of both said environment acquired data (51) and user's (1) personal data.

5. Electronic system as claimed in claim 4, **characterized in that** said user's (1) personal data are managed in a first database without external access by third parties, while said environment acquired data (51) are stored in second databases and managed anonymously by said digital platform (100) and by said third party companies.

6. Electronic system as claimed in claim 3, **characterized in that** said third party companies are able to sell (101) said environment acquired data (51) to third parties (200), such as entities and/or organizations and/or public or private companies, said third parties (200) being able to pay (201) said environment acquired data (51) to third-party companies, which convert the data obtained (102) into utility tokens (70) associated with said user (1).

7. Electronic system as claimed in at least one of the preceding claims, **characterized in that** said user (1) is recognized by said digital platform (100) by means of said software APP (11) running on said smartphone (10) and through its own credentials access.

8. Electronic system as claimed in claim 6, **characterized in that** said user (1) is able to view and display (71) said associated utility tokens (70), to request (53) and/or use (72) said utility tokens (70) for purchasing goods, for example in shops and/or e-commerce sites (80).

9. Electronic system as claimed in at least one of the preceding claims, **characterized in that** said user (1) is able to request (54) and obtain said environment data from said digital platform (100) by means of said software APP (11) of a specific place or area and to display (55) or receive (56) from said digital platform (100) service communications for said third companies and/or other partners, upon request and/or explicit acceptance by the user (1).

10. Environment monitoring method which is implemented by means of an electronic environment monitoring system as claimed in claim 1.
